**Europäisches Patentamt**

**European Patent Office**

**Office européen des brevets**

(11) Numéro de publication : **0 351 344 B1**

(12) **FASCICULE DE BREVET EUROPEEN**

(45) Date de publication du fascicule du brevet :
26.08.92 Bulletin 92/35

(51) Int. Cl.⁵ : **A61M 5/14**

(21) Numéro de dépôt : **89480075.4**

(22) Date de dépôt : **12.05.89**

(54) **Moyen de pressurisation pour poches de perfusion.**

(30) Priorité : **16.05.88 FR 8806832**

(43) Date de publication de la demande :
**17.01.90 Bulletin 90/03**

(45) Mention de la délivrance du brevet :
**26.08.92 Bulletin 92/35**

(84) Etats contractants désignés :
**AT BE CH DE ES FR GB GR IT LI LU NL SE**

(56) Documents cités :
**EP-A- 0 102 012**
**EP-A- 0 210 424**
**GB-A- 2 118 634**
**US-A- 4 090 514**

(73) Titulaire : **Antonetti, Pierre**
**7 Avenue Jean Jaurès**
**F-06540 Breil sur Roya (FR)**

(72) Inventeur : **Antonetti, Pierre**
**7 Avenue Jean Jaurès**
**F-06540 Breil sur Roya (FR)**

(74) Mandataire : **Hautier, Jean-Louis**
**Cabinet Hautier Office Méditerranéen de**
**Brevets d'Invention et de Marques 24 rue**
**Masséna**
**F-06000 Nice (FR)**

EP 0 351 344 B1

## Description

La présente invention concerne un dispositif de perfusion parentérale portable et automatique à pression d'air régularisée par un détendeur. Ce dispositif autonome peut être fixé sur le sujet ou à proximité immédiate, il fonctionne en circuit fermé, il est indépendant de la gravitation et de la pression atmosphérique.

Cette invention trouve son application dans le domaine médical et vétérinaire.

Il est un fait commun de voir brancarder un blessé grave ou un malade sous perfusion; une personne suit le brancard, portant à une certain hauteur un flacon de sérum ou de sang et sa tubulure de perfusion. Le flacon de soluté se trouve au-dessus de la veine perfusée pour permettre l'écoulement par gravité du liquide qu'il contient.

Ce procédé comporte de nombreux inconvénients auxquels plusieurs brevets ont déjà tenté de porter remèdes.

### ETAT DE L'ART ANTERIEUR.

HINCK ( U.S A 4 090 514 du 23-05-78 ) décrit une vessie, gonflable au moyen du dispositif habituel de poire à pression et de manomètre, utilisé dans les brassards à tension : l'écoulement du liquide à perfuser se faisant par le procédé connu de goutte à goutte. Le dispositif selon HINCK est destiné à être accroché verticalement en lieu et place du flacon de verre près du lit du malade. Fonctionnant par gravité, il sert surtout à accélérer le débit : il n'est pas prévu pour fonctionner dans toutes les positions.

Le dispositif de HINCK ne comprend qu'une seule poche de pression : il ne peut donc assurer la régularité parfaite de la perfusion. En effet, au fur et à mesure que le flacon à perfuser se vide, la pression du ballonnet-presseur entourant le flacon , ne peut que diminuer, ce qui se traduit par un ralentissement de la vitesse d'écoulement du liquide perfusé. Pour remédier à cet inconvénient, l'opérateur peut soit remonter la pression à l'aide de la poire, soit modifier le débit du compte-goutte, ce procédé n'est donc pas automatique et nécessite la surveillance constante d'une tierce personne.

Dans tous les cas, la régularité n'est pas assurée et demande une intervention.

L'appareil de HINCK ne permet pas d'assurer des perfusions dans une gamme fonctionnelle de pressions, celles-ci n'étant pas régulées.

Deux autres brevets LEIBINSOHN (EP A 0102 012 du 07-03-84) et ZORATTO (EP A 0 210 424 du 04-02-87) reprennent sensiblement les mêmes éléments et n'apportent pas d'éléments inventifs nouveaux par rapport à HINCK.

B. KEIME ( G.B A 2 165 312 du 09-04-86 ) décrit un dispositif avec boitier étanche contenant la poche de liquide à perfuser, raccordée à une cartouche de gaz inerte par le moyen d'un détendeur. Il s'agit d'un boitier solide non extensible, donc l'équivalent d'un dispositif à une seule poche, identique dans le principe aux brevets précédents

Les inconvénients liés au dispositif de KEIME sont les suivants :

– l'utilisation d'une cartouche de gaz inerte ne renseigne pas sur son état de remplissage,

– en l'absence de cartouche, ce dispositif est inutilisable.

– la tubulure du flacon de soluté contenu dans le boitier étanche doit traverser la paroi au moyen d'un joint ou presse étoupe soumis à la pression interne. L'étanchéité du boitier, la détérioration du joint, et les problèmes inhérents au passage de la tubulure sont un handicap.

Dans les pays du Tiers Monde, où l'approvisionnement en cartouche de gaz et l'entretien sont aléatoires, il fallait trouver un procédé manuel mieux adapté aux habitudes du Corps Médical.

Le dispositif selon l'invention se différencie complètement des dispositifs cités ci-dessus par le fait qu'il comporte :

– deux poches de pression distinctes :

. un accumulateur d'énergie ou ballonnet de pression,

. un ballonnet-presseur comprimant le flacon souple

– un moyen de gonflage, manuel ou automatique,

– un détendeur assure la liaison et la régulation de pression entre les deux poches. Il permet de réguler entre $10^4$ Pa à $4.10^4$ Pa (100 et 400 mbar), à la demande, la pression exercée sur le flacon de soluté.

Le dispositif, indépendant de la gravitation, est solidaire du sujet ou placé dans son environnement immédiat.

### Description de l'invention

Selon une forme préférentielle de réalisation de l'invention, le dispositif comprend deux enveloppes souples, non extensibles, renfermant chacune un ballonnet extensible, gonflable à l'air ou à tout autre gaz. Ces deux enveloppes sont réunies par une partie centrale qui reçoit les éléments de liaison entre les ballonnets.

La première enveloppe (1) est rectangulaire, cousue sur trois bords. Le bord libre permet d'insérer un ballonnet de la même forme que l'enveloppe et l'occupant en entier, gonflable par une pompe ou une poire (7) manuelle munie d'un manomètre (9) et une mollette d'arrêt (8). Ce ballonnet de pression (5) set d'accumulateur d'énergie. La fermeture est assurée par une fermeture éclair (10).

Une pression identique peut être assurée par une cartouche ou une bouteille de gaz sous pression.

La deuxième enveloppe (2), en forme de manchon, comporte deux parties :

– la premier partie (3) est identique à la première enveloppe (1) et contient donc un ballonnet extensible, gonflable, dénommé ballonnet-presseur (6).

– la deuxième partie (4), est constituée d'un rabat, également rectangulaire, cousu par ses bords latéraux à la première (3) déterminant un manchon dans lequel est inséré le flacon souple de soluté (11), qui se trouvera comprimé entre le rabat (4) et le ballonnet-presseur (6). Elle comporte une fenêtre (12) visualisant le flacon (11).

La partie centrale (13) du perfuseur, réunit les deux enveloppes et leur contenu. Elle supporte le détendeur (14) et un verticaliseur de compte-goutte (15).

Le détendeur (14) assure la liaison par tubulure souple entre le ballonnet de pression (5) en amont et le ballonnet-presseur (6) en aval.

Il permet de régler et de réguler la pression de 400 mbar établie dans le ballonnet de pression (5) pour obtenir dans le ballonnet-presseur (6) une pression, à la demande , de 100 à 400 mbar. Les positions peuvent être préréglées à 100, 200 et 400 mbar.

Le régulateur de pression joue un role très important car il permet de s'adapter à une pression extérieure inhabituelle (transport aérien, espace, montagne ).

Fonctionnement du dispositif

Ce dispositif se présente les deux enveloppes l'une contre l'autre comme un livre fermé, ou comme une trousse enserrant les organes de mise en pression et de fonctionnement (7-8-9-14-15). Ouvert il peut être fixé sur le sujet ou à proximité immédiate par des moyens classiques.

La mise en service consiste :

– à insérer dans l'espace du manchon le flacon souple de soluté (11),

– à gonfler le ballonnet de pression (5) à l'aide de la poire (7) aux environs de 400 mbar ou à relier à un système de mise en pression,

– à régler le détendeur (14) à la pression à laquelle on désire soumettre le ballonnet-presseur (6).

Le flacon de soluté ou de sang à perfuser, enserré dans le manchon est alors soumis à la pression du ballonnet-presseur (6).

Il reste à établir la perfusion à l'aide d'une tubulure classique et à verticaliser le compte-goutte.

La perfusion peut s'effectuer ainsi automatiquement et sans manoeuvre exterieure pendant plusieurs heures en continu.

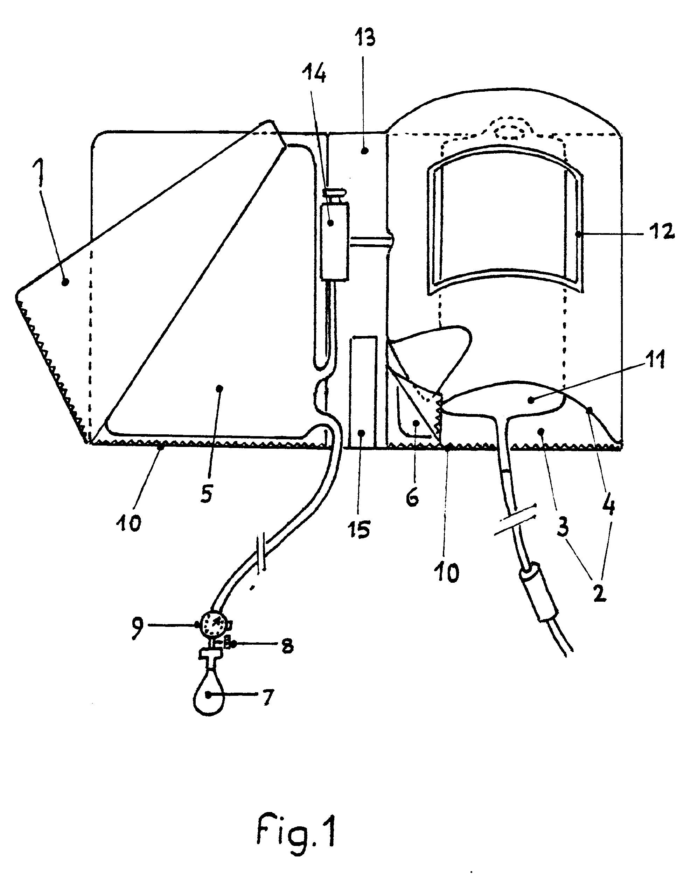

## Revendications

1) Dispositif de transfusion et de perfusion intra-veineuse et intra-artérielle, utilisable chez l'Homme et chez l'animal, solidaire du sujet et fonctionnant en circuit fermé, comportant

– une enveloppe (1), rectangulaire, fermée, souple et non extensible, contenant un ballonnet de pression (5), extensible, gonflable par un moyen (7),

– un dispositif de verticalisation (15) du compte-goutte,

Le dispositif fonctionnant automatiquement, caractérisé par :

– une seconde enveloppe (2) rectangularie, fixée à la première, souple et non extensible, contenant un ballonnet-presseur (6), extensible, gonflable, de la même forme que l'enveloppe (2) et l'occupant en entier, cousue à un rabat (4) formant ainsi un manchon destiné à recevoir le flacon de soluté (11),

– un système à deux poches de pression communiquant par l'intermédiaire d'un détendeur calibré, régulateur de pression (14), réglable, qui régularise de $10^4$Pa à $4.10^4$Pa (100 à 400) mbar, dans le ballonnet-presseur (6) la pression de $4.10^4$Pa (400 mbar) établie en amont dans le ballonnet de pression (5),

2) Dispositif selon la revendication 1, caractérisé en ce que le moyen de gonflage (7) est une pompe manuelle munie d'un manomètre (9) et d'une mollette d'arrêt (8).

3) Dispositif selon la revendication 1, caractérisé en ce que le rabat (4) du manchon comporte une fenêtre transparente (12) permettant de visualiser le contenant et le contenu du flacon de soluté (11).

## Patentansprüche

1) Vorrichtung zur intravenösen und intra-arteriellen Transfusion und Infusion, die bei Mensch und Tier verwendbar und fest mit dem Körper verbunden ist sowie im geschlossenen Kreislauf funktioniert, mit

– einer geschlossenen rechteckigen, nicht vergrößerbaren Hülle (1) aus einem weichem Material, enthaltend ein vergrößerbares Druckballonett (5), das durch ein Mittel (7) aufblasbar ist, und

– einer Vertikalisierungseinrichtung (15) für den Tropfenzähler,

wobei die Vorrichtung automatisch arbeitet, **gekennzeichnet** durch

– eine zweite rechteckige, nicht vergrößerbare Hülle (2) aus einem weichen Material, welche an der ersten Hülle befestigt ist und ein vergrößerbares, aufblasbares Druckballonett (6) enthält, das dieselbe Form aufweist wie die Hülle (2) und diese voll ausfüllt, vernäht mit einer Krempe (4) zur Bildung eines Stutzens für die Aufnahme der Flasche mit dem Lösungsstoff (11),

– ein System mit zwei Drucktaschen, welche über ein geeichtes Entspannungsventil miteinander in Verbindung stehen, und einstellbarem Druckregler (14), der den Druck von $4.10^4$Pa (400 mbar), der stromaufwärts in dem Druckballonett (5) entstanden ist, in dem Druckballonett (6) von $10^4$Pa bis $4.10^4$Pa regelt.

2.Vorrichtung nach Anspruch 1, dadurch gekennzeichnet, daß das Aufblasmittel (7) eine Handpumpe mit einem Manometer (9) und einem arretierenden Bördelrad (8) ist.

3) Vorrichtung nach Anspruch 1, dadurch gekennzeichnet, daß die Krempe (4) des Stutzens ein durchsichtiges Fenster (12) aufweist, um das Innere sowie den Inhalt der Flasche (11) mit dem Lösungsstoff sichtbar zu machen.

**Claims**

1) Apparatus for the intravenous and intra-arterial transfusion and infusion on human beings and animals, which is firmly connected to the respective organism and functions on the basis of an enclosed circuit, comprising

– an enclosed rectangular, non-extendible envelope (1) of a soft material, containing an extendible, pressure ballonet (5) inflatable by a means (7), and

– a verticalization means (15) for the dropper, the apparatus working automamtically,

**characterized** by

– a second rectangular, non-extendible envelope (2) of a soft material, which is attached to the first envelope and and contains an extendible, inflatable pressure ballonet (6) having the same form as the envelope (2) and fully occupying the same, sewn with a rim (4) for forming a connecting piece to receive the botile with the solute (11),

– a system with two pressure pockets communicating via a gauged relief valve, and an adjustable pressure regulator (14) for regulating the pressure of $4.10^4$Pa (400 mbar) generated upstream in the pressure ballonet (5) from $10^4$ Pa to $4.10^4$Pa (100 to 400 mbar) in the pressure ballonet (6).

2. The apparatus according to claim 1, characterized in that the inflating means (7) is a manual pump with a manometer (9) and a beaded locking wheel (8).

3) The apparatus according to claim 1, characterized in that the rim (4) of the connecting piece comprises a transparent window (12) so as to allow viewing the interior and the content of the bottle (11) with the solute.

Fig.1